# EUROPÄISCHE PATENTSCHRIFT

(11) **EP 2 182 896 B1**
(45) Veröffentlichungstag und Bekanntmachung des Hinweises auf die Patenterteilung: **18.11.2015**
(21) Anmeldenummer: 08801263.8
(22) Anmeldetag: 26.08.2008
(51) Int. Cl.: A61F 2/958, A61M 25/10

(54) **BALLONKATHETER MIT SCHUTZ VOR AUFFALTUNG**
CONTROLLED EXPANSION BALLOON CATHETER
CATHÉTER À BALLONNET POURVU D'UNE PROTECTION EMPÊCHANT UN DÉPLOIEMENT PRÉMATURÉ

(30) Priorität: 29.08.2007 DE 102007040868
(43) Veröffentlichungstag der Anmeldung: 12.05.2010
(73) Patentinhaber: Invatec Technology Center GmbH, 8500 Frauenfeld (CH)
(72) Erfinder: SCHELLER, Bruno, 66121 Saarbrücken (DE); SPECK, Ulrich, 10098 Berlin (DE)
(74) Vertreter: Zimmermann & Partner Patentanwälte mbB
(86) Internationale Anmeldenummer: PCT/DE2008/001456
(87) Internationale Veröffentlichungsnummer: WO 2009/026914

(56) Entgegenhaltungen:
- WO-A-95/11055
- WO-A-2007/008829
- WO-A2-03/039345
- US-A- 5 304 121
- US-A- 5 370 614
- US-A- 5 603 698
- US-A- 5 893 840
- US-A1- 2003 181 973
- US-B1- 7 105 018

## Beschreibung

Die vorliegende Erfindung betrifft wirkstoffbeschichtete Ballonkatheter mit distal montiertem Katheterballon und einem diesen umgebenden Expandierschutz, wobei der Katheterballon zum einen auch bei starker mechanischer Belastung vor vorzeitiger Auffaltung geschützt ist und zum anderen der Katheterballon nach erfolgter Dilatation eine bessere Rückfaltung aufweisen kann.

Katheter sind unterschiedlich lange Schläuche oder anderweitig leitende gestreckte Körper, proximal mit einem Griff und/oder Ansatzstück zum Anschluß unterschiedlicher Instrumente, Verlängerungen oder Behälter, distal offen oder geschlossen ohne oder mit Bauteilen unterschiedlicher Funktion. Katheter dienen der Ausübung verschiedener Eingriffe und Funktionen im Körper. Eine sehr nützliche Form der Katheter enthält im distalen Bereich einen Ballon. Der Ballon wird gewöhnlich in einem eingefalteten oder kontrahierten Zustand an den Bestimmungsort gebracht. Er kann mit geringem oder hohem Druck durch Füllen mit Flüssigkeit expandiert werden und damit der Fixierung des Katheters dienen oder der Schaffung oder Erweiterung von Hohlräumen in natürlichen Gängen, Blutgefäßen, Körperhöhlen oder auch im festen Gewebe. Die Erweiterung von Koronararterien mit Ballonkathetern wird als perkutane transluminale coronare Angioplastie (PTCA), die Erweiterung sonstiger Arterien allgemein als perkutante transluminale Angioplastie (PTA) bezeichnet. Der Ballon kann als Träger von chemischen Substanzen, Radioisotopen, Arzneistoffen, Diagnostika oder sonstigen nützlichen Materialien oder Funktionen dienen.

### Stand der Technik

Ballone mit definierten Außenmaßen bestehen aus wenig dehnbaren Materialien. Sie werden in eingefaltetem, meist eingerolltem Zustand an den Ort gebracht, an dem sie durch Insufflation mit Flüssigkeit, meist einem verdünnten Kontrastmittel, auf ihre vorbestimmte Größe aufgeweitet werden. Sehr vorteilhaft ist ein geringer Durchmesser, der durch eine feste Faltung erzielt wird. Diese Faltung ist bei den heute gebräuchlichen Ballonkathetern nicht besonders fixiert. Sie kann sich während der Handhabung, bei der Passage durch Einführschleusen und Führungskathetern lockern und sogar völlig öffnen. Nach Gebrauch wird die Flüssigkeit aus den Ballonen unter Unterdruck abgesaugt, der Ballon vermindert seinen Querschnitt, bleibt allerdings entfaltet. Vorzeitige Lockung der Falten erhöht den Ballonquerschnitt in unerwünschter Weise und führt bei beispielsweise mit Arzneimitteln beschichteten Ballonen zu einer vorzeitigen Abgabe zumindest eines Teils des Wirkstoffs. Die mangelnde Festigkeit der Faltung macht bestimmte Maßnahmen notwendig, um eine vorzeitige Abgabe der Beschichtung zu verhindern und schränkt die Auswahl der Beschichtungsmaterialien stark ein. Insbesondere sind flüssige Medien und gut wasserlösliche Wirkstoffe zur Beschichtung von Ballonkathetern bisher wenig geeignet, da diese z. B. im Blutstrom abgewaschen werden bevor der Ballon das Zielgebiet erreicht.

Ballonkatheter können mit Stents versehen werden. Stents sind röhrenförmige Metall- oder auch Kunststoffgebilde, die über die gefalteten Ballone geschoben werden und diese eng gefaltet halten bis sie in dem zu behandelnden verengten Gefäßsegment mit hohem Druck expandiert werden. Dabei weiten sich die Stents radial auf. Sie stabilisieren die verengten Blutgefäße gegen eine elastische Rückstellkraft und fixieren gleichzeitig abgelöste Teile der Gefäßwand (Dissektionen). Um diese Aufgabe zu erfüllen sind Stents durch eine möglichst hohe radiale Druckfestigkeit gekennzeichnet. Sofern Ballonkatheter mit vormontierten Stents versehen sind, gibt es keine Probleme mit einer vorzeitigen Auffaltung der Ballone.

Bei vielen Anwendungen von Ballonkathetern ist jedoch die Implantation von Stents nicht notwendig oder sogar schädlich.

Bisher sind folgende Maßnahmen bekannt, die eine vorzeitige Lockerung der Faltung verhindern oder die eingeführt wurden, um die Haftung von Substanzen an Ballonkathetern zu verbessern oder deren vorzeitige Diffusion in das umgebende Medium zu reduzieren:
Vorab montierte ballonexpandierbare Stents auf den gefalteten Ballonen halten die Faltung zusammen solange bis der Ballon am Ort der Stenose inflatiert, der Stent expandiert und gegen die Gefäßwand gedrückt wird. Der Stent verbleibt nach Deflatieren in dem Gefäß, um eine Verengung aufgrund elastischer Eigenschaften der Gefäßwand zu verhindern und ggf. abgelöste Schichten der Gefäßwand (Dissektionen) dauerhaft an die Gefäßwand zu drücken. Als Maßnahme gegen eine Verschiebung von ballonexpandierbaren Stents auf den gefalteten Ballonen wurden Schutzschläuche verwendet, die über die gesamte Katheterlänge von distal des Stents bis zum proximalen Ende des Katheters reichen (z.B. CA 2 372 820). Der Schutzschlauch wird vor Expansion des Stents zurückgezogen. Schutzschläuche verschiedener Art wurden bisher bei selbstexpandierenden Stents eingesetzt, wobei der durch den Schutzschlauch auf einen geringen Durchmesser zusammengedrückte Stent durch Zurückziehen des Schutzschicht freigesetzt wird, sich aus eigener Kraft auf einen größeren Durchmesser aufweitet und an die Gefäßwand anlegt.

According to its title, WO 2007/008829 relates to a drug delivery system.

WO 03/039345 according to its title describes a stent delivery device with embolic protection.

In EP 1 140 273 wird die Verwendung gesättigter wässriger physiologisch annehmbarer Lösungen zur lokalen Therapie mittels Kathetern vorgeschlagen. Als Maßnahmen gegen das vorzeitige Abwaschen der Lösungen von expandierbaren Kathetern wird die Verwendung von Schutzschläuchen oder an den Kathetern haftenden die Lösung absorbierenden Polymeren vorgeschlagen.

Nach USP 6 618 650 wird ein Führungskatheter als Schutz für einen mit einem Arzneistoff beschichteten und mit Stent versehenen Ballon verwendet, wobei der Ballon erst unmittelbar vor der Expansion in der Stenose von dem Führungskatheter freigesetzt wird.

Poröse Hüllen bzw. Manschetten aus bispielsweise PTFE wurden auch beschrieben, die den gefalteten Ballon umgeben. Der Arzneistoff bzw. dessen Lösung befindet sich zwischen der Ballonmembran und der Hülle und wird durch den Druck beim Expandieren des Ballons durch die Poren der Hüte herausgopresst.

US 5370814 A1 beschreibt einen Ballon mit einer viskösen Beschichtung, die durch eine dünnwandige Hülle mit vorgeformter Längsnaht vor dem vorzeitigen Ablösen geschützt ist. Beim Aufdehnen des Ballons reißt die Hülle an der vorgeformten Naht, deckt jedoch einen Längsstreifen der Getäßwand ab, so daß dieser nicht mit der viskösen Beschichtung in Kontakt kommt.

Eine weitere Version von aufplatzenden Hüllen ist In US 2002/0 151 844 beschrieben: Es handelt sich um eine Deckschicht aus unelastischem, für das Arzneimittel undurchlässigem Material, die beim Expandieren des Ballons zahlreiche Risse bekommt, durch die der Arzneistoff ausgewaschen wird.
USP 5,102,402 beschreibt Ballone mit speziellen Vertiefungen (Falzen) in der Ballonholle. In diesen Fallen können Mikrokapseln festgehalten werden. Beim Aufdehnen der Ballone öffnen sich die Falzen und geben die Mikrokapseln an die Umgebung ab.

In WO 2000/010622 A werden Ballonmaterialien mit rauen Oberflächen beschrieben. An diesen sollen Arzneistoffe haften. Zahlreiche andere Patentschriften beschreiben die Verwendung von Hydrogelen oder anderen Polymeren oder Beschichtungsmaterialien zur Verbesserung der Haftung von Arzneistoffen an Ballonoberflächen und zur Verzögerung der Freigabe von Wirkstoffen (z.B. USP 5,304,121WO 9639949, WO 9908729, USP 6,306,166, US 2005/0033417) oder die Verwendung von wenig wasserlöslichen oder öligen Beschichtungen (WO 02076509).

Ein mehrfacher Schutz vor vorzeitiger rascher Wirkstoffabgabe wird entsprechend der WO 00/21584 A durch Wahl eines schlecht wasserlöslichen Wirkstoffs, dessen Einbettung in ein Polymer und zusätzlich einen Schutzschlauch erzielt, wobei der Schutzschlauch bis über den distalen, expandierbaren Bereich des Ballonkatheters reicht.

Die bisher beschriebenen Maßnahmen beseitigen das Problem der vorzeitigen Ablösung einer Arzneistoffbeschichtung von Ballonkathetermembranen im Blut nicht. Die distal offenen Führungskatheter oder Schutzschläuche füllen sich mit Blut, das je nach den Druckverhältnissen am beschichteten Ballon vor oder zurück strömt. Die Lage des Schutzschlauches in Bezug auf den Ballon ist ggf. unsicher oder schwer kontrollierbar. Flüssige, auch hochvisköse Beschichtungen sind bei der Langzeitlagerung chemisch weniger stabil als feste Zubereitungen und schwieriger zu sterilisieren. Schließlich haben die beschriebenen abplatzenden oder reißenden Hüllen den Nachteil, daß Teile der Gefäßwand von den verbleibenden Teilen der Hüllen abgedeckt werden und somit dem Arzneimittel nicht zugänglich sind und wie im Falle der US 2002/0 151 844 Hüllenbruchstücke zur Embolisierung der Gefäße führen können.

### Beschreibung der Erfindung

Aufgabe der vorliegenden Erfindung ist es, Ballonkatheter mit distal montierten Katheterballonen bereitzustellen, welche zum einen auch bei starker mechanischer Belastung vor vorzeitiger Auffaltung der Katheterbalione geschützt sind und zum anderen nach Dilatation eine bessere Rückfaltung der Katheterballone besitzen. Weiterhin ist es Aufgabe der Erfindung, den unter anderem durch vorzeitige Auffaltung arzneimittelbeschichteter Ballone bedingten Verlust an Wirkstoffen zu minimieren.

Die Aufgabe der vorliegenden Erfindung wird durch die Lehre des unabhängigen Patentanspruches gelöst. Bevorzugte Ausführungsformen sind in den abhängigen Patentansprüchen, den Figuren, der Beschreibung und den Beispielen offenbart.

Problem ist, dass Ballonkatheter mit distal montierten Katheterballonen, die eine weitgehend vorgeformte Größe im Hinblick auf Durchmesser und Länge enthalten wie sie z.B. bei der perkutanten transluminalen Angioplastie verwendet werden, bei mechanischer Belastung zu einer vorzeitigen Auffaltung der Katheterballone neigen und diese verhindert werden soll.

Ein weiteres Problem besteht darin, dass die Rückfaltung der Katheterballone unzureichend ist und nach der Dilatation aufgrund des erzeugten Unterdrucks nur eine teilweise Rückfaltung möglich ist, so dass ein Katheterballon in der Regel bei der Entfernung aus dem Körper einen größeren Durchmesser hat als beim Einführen in den Körper, was beispielsweise dann zu Komplikationen führt, wenn verengte Gefäßabschnitte passiert werden müssen. Dies trifft beispielsweise dann zu, wenn der Katheterballon durch einen bereits gesetzten Stent geführt werden muss.

Somit betrifft die vorliegende Erfindung wirkstoffbeschichtete Ballonkatheter, welche einen Expandierschutz und einen wirkstoffbeschichteten Katheterballon umfassen, wobei der Expandierschutz bis zur Expansion den Katheterballon vor vorzeitiger Aufweitung des komprimierten Katheterballons schützt und ggf. nach erfolgter Expansion die Rückfaltung des Katheterballons unterstützen kann.

Dies kann grundsätzlich auf zwei Arten bewerkstelligt werden. Einmal indem eine starre, weitgehend starre oder feste Schutzhülle über den Katheterballon geschoben wird, welche so lange ein Aufweiten des Katheterballons und den Flüssigkeitsaustausch mit der Umgebung verhindert wie sie sich über dem Katheterballon befindet. Derartige Schutzhüllen sind vorzugsweise als massives Rohr oder massiven Schlauch ausgestaltet und umgeben vorzugsweise den Katheterballon entlang seiner gesamten Länge. Sie enthalten bevorzugt distal einen Verschluß zur Verhinderung oder Begrenzung des Blutaustauschs mit dem Lumen der Schutzhülle, durch den sich der Ballon des Katheters schieben lässt. Ein solcher Verschluß kann ein Ventil mit beispielsweise 2-5 Ventillippen bestehen, einer perforierten oder perforierbaren dünnen Membran oder auch einem löslichen Verschlußmaterial. Eine andere Möglichkeit besteht in einem elastischen Expandierschutz, der vorzugsweise den Katheterballon, d.h. die Oberfläche des Katheterballons nicht vollständig abdeckt und vorzugsweise sogar nur zu einem geringen Teil abdeckt und somit vorzugsweise eine lückenhafte Struktur aufweist. Dieser elastische Expandierschutz wird bei der Dilatation zusammen mit dem Katheterballon aufgeweitet. Zusätzlich zu seiner Elastizität weist dieser Expandierschutz eine Rückstellkraft auf, welche bei der nachfolgenden Deflation des Katheterballons diesen weitgehend in seine ursprünglich Größe zurückbringt, d.h. weitgehend den ursprünglichen Durchmesser des Katheterballons vor der Dilatation wiederherstellt.

Weitgehend die ursprüngliche Größe bzw. den ursprünglichen Durchmesser wiederherstellen bedeutet, dass der Durchmesser des Katheterballons nach der Expansion und Deflation maximal 30%, bevorzugt maximal 20%, weiter bevorzugt maximal 10%, noch weiter bevorzugt maximal 5% größer ist als vor der Expansion, d.h. der Katheterballon ist beim Entfernen aus dem Körper maximal die vorgenannten Prozentzahlen größer bzw. dicker als beim Einführen in den Körper.

Grundsätzlich ist aber der Durchmesser des Katheterballons mit Expandierschutz nach Expansion und Deflation kleiner als bei einem Katheterballon ohne Expandierschutz nach Expansion und Deflation.

Es sei angemerkt, dass die Begriffe "Expansion", "Inflation", "Dilatation", "Insufflation" oder "Aufweitung" denselben Sachverhalt bezeichnen, nämlich die Ausdehnung bzw. das Aufblasen des Katheterballons durch Anlegen eines Drucken von Innen, d.h. im Inneren des Katheterballons, was in der Regel durch Befüllung des Katheterballons mit einem Kontrastmittel geschieht.

Die Begriffe "Deflation", "Rückfaltung" oder "Komprimieren" bezeichnen hingegen den umgekehrten Vorgang, nämlich das Zusammenziehen bzw. Entleeren des Katheterballons durch Anlegen eines Unterdrucks von Innen, d.h. im Inneren des Katheterballons, was in der Regel durch Absaugen des Kontrastmittels geschieht.

Vorzugsweise wird der Expandierschutz nach der Deflation des Katheterballons mit diesem wieder aus dem Körper entfernt. Somit ist der Expandierschutz vorzugsweise mit dem Katheterballon fest oder beweglich so verbunden, dass sich beim Entfernen des Katheterballons aus dem Körper der Expandierschutz um den Katheterballon befindet.

Des weiteren weist der Expandierschutz vorzugsweise am distalen Ende oder am proximalen Ende oder am distalen Ende und am proximalen Ende einen röntgendichten Marker auf. Bei einem entlang der Katheterballonlängsachse translationsbeweglich gelagerter Expandierschutz zeigen die Röntgenmarker am Katheterballon sowie am Expandierschutz die relative Position von Expandierschutz zu Katheterballon an. Dadurch kann im bildgebenden Verfahren, z.B. Röntgenverfahren ein Arzt die Position von Katheterballon und Expandierschutz erkennen und beurteilen, wie weit der Expandierschutz den Katheterballon in Richtung der Längsachse des Katheters bzw. des Katheterballons noch umgibt.

Als Expandierschutz kann beispielsweise ein perforierter oder durchgängiger Schutzschlauch dienen. Ein durchgängiger, d.h. massiver Schutzschlauch in Form eines flexiblen Rohres ist dann gegenüber dem perforierten Schutzschlauch bevorzugt, wenn sich im Inneren des Schutzschlauchs ein beschichteter Katheterballon, mit oder ohne vormontiertem beschichtetem oder unbeschichtetem Stent oder ein unbeschichteter Katheterballon mit einem vormontierten beschichteten Steht befindet. Nicht perforierte Schutzschläuche werden bevorzugt derart gestaltet, daß Blut oder andere Körperflüssigkeiten nicht in das Lumen des Schlauches eindringen können oder zumindest ein Flüssigkeitsaustausch deutlich behindert ist. Dazu wird beispielsweise distal ein Ventil vorgesehen. Als distaler Ventilmechanismus ist beispielsweise eine lippen- oder klappenförmige Gestaltung des distal verjüngten und seitlich geschlitzten Schlauches geeignet. Dabei kann es sich um 2 Klappen, aber auch um 3 oder mehr Klappen handeln. Der Schlauch kann distal auch mittels pharmazeutisch und physiologisch unbedenklicher, löslicher Substanzen verschlossen werden. Beispiele für derartige Substanzen sind endogene Substanzen wie Zucker oder Aminosäuren, aber auch Polysaccharide, Kontrastmittel wie jodierte Röntgenkontrastmittel jeweils in fester Form. Der Verschluß mit Kontrastmitteln hat den Vorteil daß man dessen Anwesenheit während der Intervention laufend sichtbar machen kann. Alternativ oder zusätzlich kann der Schlauch proximal mit einem Ventil oder Verschluß versehenen werden, der den Raum zwischen dem Katheterschaft und dem Schlauch abdichtet. Geeignete Verschlußmechanismen sind flexible Membranen aus beispielsweise Latex oder Silikonkautschuk oder adjustierbare Verschlüsse mit Quetschdichtungen wie sie an Y-Adaptatoren für arterielle Einführschleusen verwendet werden. Das Lumen des Schutzschlauches kann mit einer geringen Menge eines bevorzugt trockenen Gerinnungshemmers wie beispielsweise Heparin oder Zitrat beschichtet oder versehen sein.

Der Begriff "vormontiert" bezeichnet einen auf dem Katheterballon aufgesetzten und fest an der Katheterballonoberfläche anliegenden Stent. Dieser kann z.B. im Falle eines Stahlstents zur Expansion auf einen größeren Durchmesser Kraft erfordern oder z.B. im Falle eines Nitinolstents nur durch eine entsprechende Kraft (beispielsweise einen Hüllschlauch) daran gehindert werden, sich spontan auf einen größeren Durchmesser aufzuweiten.

Der Begriff "beschichtet" oder "Beschichtung" meint in der Regel eine wirkstofffreisetzende Beschichtung, welche aus mindestens einem Wirkstoff selbst ohne weitere Hilfsstoffe oder aus mindestens einem Wirkstoff in einem Trägersystem mit weiteren Hilfsstoffen wie polymeren, oligomeren oder nicht-polymeren Stoffen besteht.

Dieser perforierte oder durchgängige Schutzschlauch kann, wenn der Katheterballon an seinem bestimmten Wirkort angelangt ist, von seinem proximalen Ende aus in proximale Richtung bewegt werden, wobei die Bewegung in proximale Richtung den Katheterballon teilweise oder völlig freigesetzt.

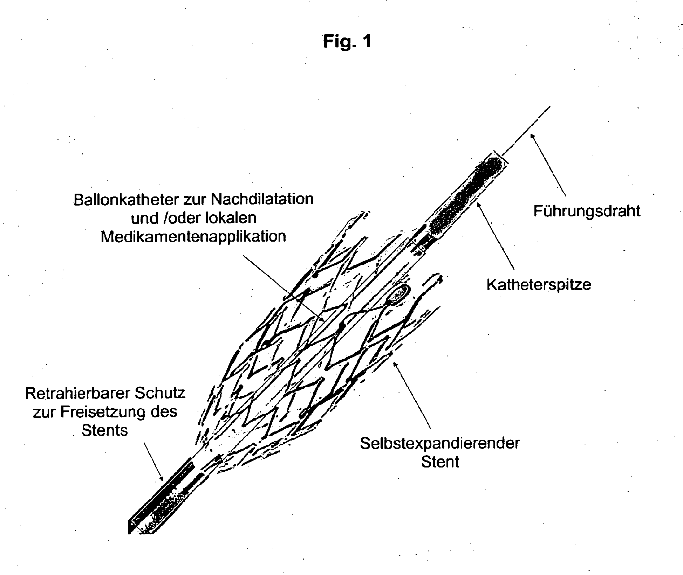

Das "distale" Ende des Katheters bzw. Katheterballons ist an der Katheterspitze (s. Fig. 1) und dementsprechend ist das "proximale" Ende, das dem distalen Ende gegenüberliegende Ende. Eine Bewegung in proximale Richtung entspricht also einer Bewegung in die Richtung, in welcher der Katheterballon wieder aus dem Körper entfernt wird.

Erfindungsgemäß kann jedoch der Expandierschutz in Form des Schlauches nicht in distale Richtung bewegt werden, wenn sich der Expandierschutz vollständig über dem wirkstoffbeschichteten Katheterballon befindet. Wird jedoch vor der Expansion des Katheterballons an seinem Bestimmungsort der Schlauch in proximale Richtung vom Katheterballon entfernt, wodurch der Katheterballon ganz oder zumindest teilweise freigesetzt wird, so kann danach der Schlauch wieder in distale Richtung translatorisch bewegt werden, bis hin zu seiner Ausgangsposition vor der Dilation bzw. während des Einführens des Katheterballons in den Körper.

Durch diese Bewegung in distale Richtung bis zu dem Punkt, wo der Schlauch den Katheterballon wieder weitgehend vollständig umgibt, erfolgt eine Unterstützung der Rückfaltung des Katheterballons, wodurch ein kleinerer Querschnitt bzw. Durchmesser erreicht wird, als wenn der Schlauch nicht wieder über den Katheterballon geschoben würde.

Somit schützt dieser Expandierschutz in Form eines perforierten oder durchgängigen Schlauches nicht nur den wirkstoffbeschichteten Katheterballon mit oder ohne Stent bei der Einführung des Katheters in den Körper bzw. in das Gefäß oder Hohlorgan oder den Körperdurchgang, sondern unterstützt auch die Rückfaltung wieder zu einem Durchmesser annähernd dem zum Zeitpunkt der Einführung des Katheters. Bei dieser bevorzugten Ausführungsform ist der Schlauch vorzugsweise über die gesamte Länge des Katheterballons angebracht. Der Schutzschlauch wird unmittelbar bevor der Katheterballon inflatiert werden soll um die Ballonlänge oder mehr zurückgezogen. Wird der Schlauch nicht um die gesamte Ballonlänge zurückgezogen so wird nur ein Teil des Ballons aufgedehnt. Zur Kontrolle der Position des Schutzschlauches kann dieser wie oben erwähnt im distalen Bereich röntgendichte Marker enthalten, deren relative Position zu den ebenfalls röntgendichten Markern am Ballonschaft vor Expansion des Ballons bestimmt wird. Auf diese Weise kann der Arzt unmittelbar vor Gebrauch die nutzbare Ballonlänge millimetergenau festlegen.

Auf der Oberfläche des Katheterballons und unter dem Expandierschutz in Form des perforierten oder durchgängigen Schlauchs kann sich ein Stent befinden, wobei vorzugsweise selbstexpandierende Stents wie z.B. Nitinol-Stents verwendet werden, wobei selbstexpandierende Stents jedoch nicht zwingend sind.

Die bekannten Katheter zur Implantation von selbst expandierenden Stents enthalten - wie oben beschrieben - einen Schutzschlauch, dessen Aufgabe es ist, den entlang der Katheterachse auf einen geringen Querschnitt komprimierten Stent zunächst in diesem Zustand zu fixieren, bis Katheter mit Stent und Schutzschlauch in die Position gebracht sind, in der die Behandlung erfolgen soll. Wenn sich der Ballon mit dem Stent in dem z. B. stenotischen Bereich des Blutgefäßes befindet wird zunächst der Schutzschlauch zurückgezogen und damit der Stent freigesetzt. Dieser legt sich an die noch immer verengte Gefäßwand an. Da die Rückstellkraft des selbstexpandierenden Stents aber nur gering ist, wird häufig nicht der gewünschte Durchmesser des Gefäßlumens erreicht. In diesen Fällen wird der ursprünglich verwendete Katheter aus dem Blutgefäß entfernt und es wird ein zusätzlicher Ballonkatheter ohne Stent eingeführt. Anschließend wird der Ballon mit hohem Druck unter Verwendung eines Kontrastmittels soweit expandiert, dass der ursprüngliche bzw. gewünschte Gefäßdurchmesser erreicht wird. Der Ballon kann bei diesem Vorgang einen Arzneistoff auf die Gefäßwand übertragen. Durch den Arzneistoff wird eine unerwünschte Verdickung der Gefäßwand im Heilungsprozess und danach vermieden und das Gefäß langfristig offen gehalten.

Erfindungsgemäß lässt sich dieser Vorgang wesentlich vereinfachen, zuverlässiger und wirksamer gestalten, wobei der den Stent komprimierende Schlauch als Expandierschutz auch den Wirkstoff vor der vorzeitigen Freisetzung bewahrt. Das neuartige System besteht aus dem gebräuchlichen Katheter, der distal mit einem üblichen PTCA oder PTA-Ballon versehen ist. Auf dem gefalteten Ballon ist vorzugsweise ein selbstexpandierender Stent, z.B. aus Nitinol montiert, der durch den Schlauch auf einen engen Durchmesser komprimiert wird. Der Schlauch kann vom proximalen Ende her zurückgezogen werden, mindestens bis der Stent in der gesamten Länge freigesetzt ist. Der Katheterballon ist mindestens so lang wie der Stent, in einer bevorzugten Ausführungsform an beiden Enden 1 bis 10 mm länger, in einer besonders bevorzugten Form 5 - 10 mm länger.

Wesentlicher Vorteil des Systems ist es, dass mehrere Arbeitsgänge ohne Katheterwechsel möglich sind und ein optimales initiales und Langzeitergebnis entsteht, rascher, kostengünstiger und in besserer Qualität als wenn die vorbekannten Katheter nacheinander angewandt werden.

Somit können die erfindungsgemäßen Ballonkatheter die Implantation von Stents ermöglichen, erfordern diese jedoch nicht. Die hierin beschriebenen neuartigen Ballonkatheter können mit beliebigen Materialien beschichtet werden, wobei die Beschichtungen durch den Expandierschutz, insbesondere den massiven bzw. nicht perforierten oder durchgängigen Schlauch vor einer vorzeitigen Freigabe bei der Handhabung und auf dem Weg zu dem Zielgewebe geschützt werden.

Des weiteren betrifft die vorliegende Erfindung wirkstoffbeschichtete Ballonkatheter umfassend einen festen und/oder schlauchförmigen Expandierschutz mit darin befindlichem selbstexpandierenden Stent und umfassend einen Katheterballon, wobei der Expandierschutz bis zur Freisetzung den Stent vor vorzeitiger Aufweitung und den Ballon vor vorzeitigem Verlust von Beschichtungsmaterial schützt.

Ferner betrifft die vorliegende Erfindung wirkstoffbeschichtete Ballonkatheter umfassend einen schlauchförmigen und/oder festen Expandierschutz mit Abdichtung des Raumes zwischen Katheterschaft und Schutzschlauch gegen das Eindringen von Körperflüssigkeiten und einen Katheterballon, wobei der Expandierschutz bis zur Expansion den Katheterballon vor vorzeitiger Aufweitung und vorzeitigem Verlust von Beschichtungsmaterial schützt, wobei der Expandierschutz optional einen selbstexpandierenden Stent enthalten oder umfassen kann.

In einer anderen bevorzugten Ausführungsform wird der Ballon durch ein elastisches röhren- oder ringförmiges Maschen-, Netz-, Spiral- oder Flechtwerk oder Gitter zusammengehalten. Solche Strukturen sind auch in US 2006/ 0085065 beschrieben, werden dort allerdings mit Wirkstoffen beladen und als Träger derselben in Verbindung mit vormontierten Stents auf Ballonkathetern verwendet. Somit betrifft die vorliegende Erfindung des weiteren Ballonkatheter mit einem Expandierschutz vorzugsweise in Form eines flexiblen Bandes, eines flexiblen Netzes, eines flexiblen Gitters, einer flexiblen Folie oder einer flexiblen Spirale.

Erfindungsgemäß übt das flexible Netz, das flexible Gitter, die flexible Folie oder die flexible Spirale bei der Expansion des Katheterballons eine entgegengesetzte zur Längsachse des Katheterballons hin wirkende Kraft aus. Diese Kraft ist nicht so stark, dass der Katheterballon wesentlich an der Expansion gehindert wird, d.h. diese durch den Expandierschutz in Form von Netz, Gitter, Folie, Spirale oder einem anderen regelmäßig oder unregelmäßig lückenhaften Geflecht ausgeübte Rückstellkraft ist kleiner als der im Inneren des Katheterballons erzeugte Druck, so dass sich der Expandierschutz zusammen mit dem Katheterballon auf den gewünschten Durchmesser ausdehnt. Bei der Deflation des Katheterballons wirkt die Rückstellkraft des Expandierschutzes jedoch in die gleiche Richtung wie der im Katheterballon erzeugte Unterdruck und trägt zu einer effektiveren und besseren Rückfaltung des Katheterballons bei.

Vorzugsweise wird der Katheterballon wieder auf einen Durchmesser gebracht, welcher maximal 30%, bevorzugt maximal 20%, weiter bevorzugt maximal 10%, noch weiter bevorzugt maximal 8%, noch weiter bevorzugt maximal 6% und noch weiter bevorzugt maximal 5% größer ist als vor der Expansion, d.h. der Katheterballon ist beim Entfernen aus dem Körper maximal die vorgenannten Prozentzahlen größer bzw. dicker als beim Einführen in den Körper.

Grundsätzlich ist aber der Durchmesser des Katheterballons mit Expandierschutz in Form von Netz, Gitter, Folie, Spirale oder einem anderen regelmäßig oder unregelmäßig lückenhaften Geflecht nach Expansion und Deflation kleiner als bei einem Katheterballons ohne einen solchen Expandierschutz nach Expansion und Deflation.

Beim Expandieren des Ballons folgt das Maschenwerk der Ballonwand in der Ausdehnung. Beim Deflatieren des Ballons kann das den gefalteten Ballon umgebende Material wieder annähernd den gleichen geringen Durchmesser wie vor der Expansion annehmen. Der wesentliche Unterschied zu den zur Stützung der Gefäßwand eingesetzten Stents ist in diesem Falle, daß das röhren- oder gitterförmige, den Ballon umgebende Material nachdem es seine Funktion erfüllt hat sich wieder auf den annähernd gleichen geringen Durchmesser zusammenzieht, den es hatte, bevor der Ballon expandiert wurde. Das Material wird nach der Gefäßerweiterung mit dem Ballon wieder aus dem Gefäß entfernt. Geeignete Materialien sind elastische, reißfeste Kunststoffe wie z.B. Latex oder elastische Metalle mit oder ohne Formgedächtnis wie z. B. Stahl oder Nitinol. Da nur eine geringe Zugbelastbarkeit und keine Druckfestigkeit gefordert wird, sind deutlich geringere Materialstärken als bei den Stents erforderlich. Auch sind die Anforderungen an Biokompatibilität geringer, da die Materialien nicht im Körper verbleiben.

In einer bevorzugten Variante sind die elastischen Materialien fest mit dem Katheter bzw. dessen Schaft verbunden. Eine ebenfalls bevorzugte Ausführungsform betrifft ein Maschengeflecht oder eine andere den Ballon umgebende und vor Lockerung der Faltung schützende Struktur mit Formgedächtnis wie es z. B. Nitinol aufweist, wobei die stabile Form jedoch im Gegensatz zu den selbst expandierenden Stents nicht die Form mit dem größeren Querschnitt ist sondern die Form mit dem engen Durchmesser. Bei Balloninsufflation mit deutlich erhöhtem Druck wird die Form aufgeweitet. Bei Deflation nimmt die Struktur von selbst wieder den engen Durchmesser an und ist durch die Einführungsschleuse des Katheters mit diesem wieder zu entfernen.

In einer weiteren Ausführungsform kann das die Faltung des Ballons während der Handhabung schützende Material in expandiertem Zustand an der Gefäßwand haften bleiben. Der wesentliche Unterschied zu den gebräuchlichen Stents ist in letzterem Falle die geringe radiale Stärke, die zwar ausreicht zu verhindern, daß sich das Material wieder von der Gefäßwand ablöst aber die Gefäßwand selbst nicht stützt. Dies erlaubt die Wahl geringerer Materialstärken als sie bei Stents Verwendung finden und die Verwendung einer Vielzahl auch mechanisch weniger fester jedoch biokompatibler Materialien. Besonders bevorzugt sind bioabbaubare Materialien.

Beispiele für bioabbaubare Materialien sind:
Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-öne), Poly-p-dioxanone, Polyanhydride, Polymaleinsäureanhydride, Polyhydroxymethacrylate, Fibrin, Polycyanoacrylate, Polycaprolactondimethylacrylate, Poly-ß-Maleinsäure Polycaprolactonbutylacrylate, Multiblockpolymere aus Oligocaprolactondiole und Oligodioxanondiole, Polyetherestermultiblockpolymere, PEG, Polybutylenterephtalat, Polypivotolactone, Polyglycolsäuretrimethylcarbonate, Polycaprolactonglycolide, Poly-γ-ethylglutamat, Poly(DTH-Iminocarbonat), Poly(DTE-co-DT-carbonat), Poly(Bisphenol A-iminocarbonat), Polyorthoester, Polyglycolsäuretrimethylcarbonate, Polytrimethylcarbonate Polyiminocarbonate, Poly(N-vinyl)-pyrrolidon, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxid-propylenoxid, weiche Polyurethane, Polyetherester, Polyethylenoxid, Polyalkenoxalate, Polyorthoester sowie deren Copolymere, Lipide, Carrageenane, Fibrinogen, Stärke, Kollagen, natürliche und synthetische Polyaminosäuren, Zein, modifiziertes Zein, Polyhydroxyalkanoate, Pectinsäure, Actinsäure, modifiziertes und unmodifiziertes Fibrin, Casein, Carboxymethylsulfat, Albumin, Hyaluronsäure, Heparansulfat, Heparin, Chondroitinsulfat, Dextran, β-Cyclodextrine, Copolymere aus PEG und Polypropylenglycol, Gummi arabicum, Guar, Gelatine, Collagen, Collagen-N-Hydroxysuccinimid, Lipide, Phospholipide, Modifikationen und Copolymere und/oder Mischungen der vorgenannten Substanzen.

In einer weiteren Ausführungsform kann der Katheterballon durch ein beispielsweise spiralförmig umlaufendes dünnes Band oder einen umlaufenden Faden oder ein Netz vor dem Auffalten gesichert werden (bezüglich der Form siehe ebenfalls US 2006/ 0085065). Andere Führungen des Fadens sind ebenfalls möglich. Faden, Band oder Netz sind proximal und/oder distal an dem Katheter befestigt. Sie können an beliebiger Stelle im Verlauf, bevorzugt in der Ballonmitte oder im Falle eines Netzes an mehreren Verbindungen eine oder mehrere Sollbruchstellen enthalten. Beim Expandieren der Ballone reißt der Faden ggf. an dieser Stelle. Faden oder Band können jedoch auch aus dehnbarem Material bestehen oder ein dehnbares Maschenwerk formen. Ein wesentlicher Unterschied zu der in US 5370614 beschriebenen Hülle ist die geringe Abdeckung der Ballonoberfläche. Beim Expandieren kommt es daher zu einem Kontakt zwischen Ballon und Gefäßwand, der nahezu die gesamte Ballonoberfläche betrifft. Als Materialien sind Kunst- und Naturstoffe von zum Fixieren der Ballonfaltung ausreichender mechanischer Festigkeit oder Metalle oder Materialgemische oder Verbundmaterialien bestehend aus mehreren Schichten verschiedener Materialien geeignet. Bevorzugt sind biokompatible Materialien ohne oder mit sehr geringer thrombogener Wirkung.

Da bei allen Anwendungen nur geringe Kräfte erforderlich sind, um den erwünschten Zweck - Vermeidung der vorzeitigen Lockerung der Ballonfalten - zu erzielen, sind extrem geringe Materialstärken ausreichend, die Ihrerseits den Querschnitt des gefalteten Ballons nur minimal vergrößern, im Vergleich zu einem nach mechanischer Belastung gelockerten Faltung sogar vermindern. Unter minimaler Vergrößerung wird eine Zunahme des Durchmessers um ≤200 µm verstanden, bevorzugt um ≤100 µm, weiter bevor um ≤ 50µm und am meisten bevorzugt ist, wenn sich der Durchmesser gar nicht vergrößert. In letzterem Falle ist die Querschnittverminderung des Ballons durch die Krafteinwirkung des Expandierschutzes größer als dessen Materialstärke.

Eine Sicherung der Ballonschutzhüllen oder Netze gegen Änderung der Lage relativ zum Ballon und gegen Verlust auf dem Weg zu dem stenosierten Gefäß kann durch ringförmige enge Strukturen proximal und/oder distal des Ballons erfolgen oder durch eine Verbindung zu dem proximalen Griff.

Der Katheterballon ist ferner mit einem oder mehreren Wirkstoffen optional zusammen mit weiteren Hilfsstoffen beschichtet. In einer bevorzugten Form enthält der Ballon einen oder mehrere vorzugsweise gut wasserlösliche oder jedenfalls hydrophile Wirkstoffe in löslicher oder wenig löslicher Form, in einer anderen bevorzugten Form finden besonders lipophile Wirkstoffe Verwendung. Die Hilfsstoffe können gut oder schlecht wasserlöslich sein. Wirkstoff bzw. Wirkstoffe und ggf. Hilfsstoffe können sich auf der Ballonmembran befinden, aber auch zwischen oder auf den Stentstreben. Der Katheterballon als auch der Stent können mit einem Wirkstoff oder Wirkstoffgemisch und optional weiteren Hilfsstoffen beschichtet sein, wobei sich auf dem Katheterballon derselbe Wirkstoff wie auf dem Stent oder auch ein anderer Wirkstoff befinden kann. Durch Wahl verschiedener Wirkstoffe für Stent und Katheterballon als auch die Wahl verschiedener Hilfsstoffe für die Stentbeschichtung als auch die Ballonbeschichtung lassen sich verschiedene Wirkstoffe und Wirkstofffreisetzungssysteme und damit verschiedene Freisetzungskinetiken miteinander geschickt kombinieren. Auf dem Katheterballon kann beispielsweise vorzugsweise ein reiner Wirkstoff, vorzugsweise ein hydrophiler Wirkstoff optional zusammen mit einem Hilfsstoffs, vorzugsweise einem nicht-polymeren Hilfsstoff und vorzugsweise einem hydrophilen Hilfsstoff wie einem Kontrastmittel verwendet werden, so dass sich eine schnelle bis spontane Freisetzungskinetik für den Wirkstoff auf dem Katheterballon ergibt. Auf dem Stent kann hingegen derselbe oder ein anderer Wirkstoff in ein polymeres Trägersystem eingebettet werden, so dass sich für den Wirkstoff auf dem Stent eine verzögerte Freisetzung bis hin zu einer Langzeitfreisetzung ergibt. Dadurch können Spontanfreisetzung und Langzeitfreisetzung miteinander kombiniert werden. Zudem besteht die Möglichkeit, den Stent mit einem polymeren Träger enthaltend mindestens einen Wirkstoff für eine verzögerte Freisetzung zu versehen und diese polymere Trägerschicht dann wiederum mit einer reinen Wirkstoffschicht eines anderen oder vorzugsweise desselben Wirkstoffs für eine schnelle Freisetzung zu versehen. Dadurch wird auf dem Stent ein System erreicht, welches die spontane als auch die verzögerte Freisetzung des mindestens einen Wirkstoffs ermöglicht und welche zusammen mit einem beschichteten Katheterballon eine vollflächige Versorgung der Gefäßwand mit Wirkstoff bei spontaner Freisetzung garantiert.

Des weiteren ist bevorzugt, wenn der Katheterballon länger ist als der darauf vormontierte Stent, d.h. der Katheterballon über das distale und proximale Ende des Stents hinausreicht. Zudem ist bevorzugt, wenn der Katheterballon auch in den über die Länge des Stents hinausreichenden Bereichen mit einem Wirkstoff und optional mindestens einem Hilfsstoff beschichtet ist, so dass bei der Stentimplantation auch die an den Enden des Stents angrenzenden Gefäßbereiche ausreichend mit Wirkstoff versorgt werden.

Bevorzugt sind trockene, pastöse wie auch zähflüssige Beschichtungen. Die Dosis der Wirkstoffe richtet sich nach deren Wirkstärke; bevorzugte Dosierungen liegen zwischen 0.1 und 10 µg/mm² Ballonoberfläche was der behandelten Gefäßwandinnenfläche entspricht. Das System ist geeignet, auch höhere Dosierungen zu verwenden.

Der Katheterballon kann mit reinem Wirkstoff, einem Wirkstoffgemisch oder mindestens einem Wirkstoff zusammen mit mindestens einem Hilfsstoff im expandierten als auch im komprimierten Zustand als auch gezielt unter den Falten oder auf der gesamten Oberfläche nur teilweise oder vollflächig beschichtet werden.

Der Expandierschutz entspricht zudem vorzugsweise in Durchmesser und Länge weitgehend dem zusammengefalteten oder vorgeformtem Katheterballon und erstreckt sich vorzugsweise zumindest über den beschichteten Bereicht. Der Expandierschutz selbst ist jedoch vorzugsweise unbeschichtet.

Der Katheterballon kann ferner einen vormontierten Stent aufweisen oder enthalten, der nach Expansion des Katheterballons wieder einen geringeren Querschnitt bzw. wieder einen geringeren Durchmesser annimmt und mit dem Ballonkatheter aus dem Körper entfernt werden kann.

Unter dem Begriff "aus dem Körper entfernen" wird verstanden "aus dem Gefäß, Gewebe, Hohlorgan oder Körperdurchgang" entfernen.

Wird ein Expandierschutz in Form von Netz, Gitter, Folie, Spirale oder einem anderen regelmäßig oder unregelmäßig lückenhaften Geflecht verwendet, so kann ein Stent auf diesen Expandierschutz gekrimpt oder aufgesetzt sein, der dazu bestimmt ist, nach der Dilatation am Bestimmungsort zu verbleiben. Als Expandierschutz kann aber auch der Stent selbst dienen, wobei der Schutz vor Expansion durch den Stent bewirkt wird. Als Stents können die herkömmlichen biostabilen oder auch bioabbaubaren Stents aus Metall, Metalllegierung oder Kunststoffen eingesetzt werden. Ferner können Stents verwendet werden, welche nach Implantation keine relevante Stützfunktion ausüben.

Ferner kann der Katheterballon als auch der Stent mit einem Wirkstoff, Wirkstoffgemisch oder einem Gemisch aus mindestens einem Wirkstoff mit mindestens einem Hilfsstoff beschichtet sein. Die Beschichtung von Ballonen und/oder Stents sind in zahlreichen Patentschriften und Publikationen beschrieben, z.B. EP 1372737, WO 2004028582, WO2004022124, WO2004006976, DE 10 2004 046244.

Als Wirkstoffe eignen sich antiproliferative, antiinflammatorische, antiphlogistische, antihyperplastische, antineoplastische, antimitotische, zytostatische, zytotoxische, antiangiogene, antirestenotische, mikrotubuli-inhibierende, antimigrative oder antithrombotische Wirkstoffe.

Beispiele für geeignete Wirkstoffe sind:
Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-All, Aristolochsäure, Arsen und arsenhaltige Oxide, Salze, Chelate und organische Verbindungen, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Biolimus, Bisparthenolidin, Bleomycin, Bombrestatin, Boswellinsäuren und ihre Derivate, Bruceanole A, B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvästatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1 a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Wismuth und Wismuthverbindungen oder -chelate, Irinotecan, Topotecan, Hydroxycarbamid, Miltefosin, Pentostatin, Pegasparase, Exemestan, Letrozol, Formestan, SMC-Proliferation-Inhibitor-2ω, Mitoxanthrone, Mycophenolatmofetil, c-myc-Antisense, b-myc-Antisense, ß-Lapachon,Podophyllotoxin, Podophyllsäure-2-ethylhydrazid, Molgramostim (rhuGM-CSF), Peginterferon α-2b, Lanograstim (r-HuG-CSF), Macrogol, Selectin (Cytokinantagonist), Cytokininhibitoren, COX-2-Inhibitor, NFkB, Angiopeptin, die Muskelzellproliferation hemmende monoklonale Antikörper, bFGF-Antagonisten, Probucol, Prostaglandine, 1-Hydroxy-11-Methoxycanthin-6-on, Scopolectin, NO-Donatoren, Pentaerythrityltetranitrat, Syndnoeimine, S-Nitrosoderivate, Tamoxifen, Staurosporin, ß-Estradiol, α-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Paclitaxel, Derivate von Paclitaxel, 6-α-Hydroxy-Paclitaxel, 2'-Succinylpaclitaxel, 2'-Succinylpaclitaxeltriethanolamin, 2'-Glutarylpaclitaxel, 2'-Glutarylpaclitaxeltriethanolamin, 2'-O-Ester von Paclitaxel mit N-(Dimethylaminoethyl)glutamid, 2'-O-Ester von Paclitaxel mit N-(Dimethylaminoethyl)glutamidhydrochlorid, Taxotere, Kohlensuboxids (MCS), macrocyclische Oligomere von Kohensuboxid, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, β-Sitosterin, Myrtecain, Polidocanol, Nonivamid, Levomenthol, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, in Virenüberträger inkorporierte Nukleinsäuren, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin, Tobramycin, Gentamycin, Penicilline wie Dicloxacillin, Oxacillin, Sulfonamide, Metronidazol, Enoxoparin, desulfatiertes und N-reacetyliertes Hepacin, Gewebe-Plasminogen-Aktivator, GpIIb/IIIa-Plättchenmembranrezeptor, Faktor Xa-Inhibitor Antikörper, Heparin, Hirudin, r-Hirudin, PPACK, Protamin, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon a, ß und γ, Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide wie Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Hydroxyanopterin, Protoanemonin, Cheliburinchlorid, Sinococulin A und B, Dihydronitidin, Nitidinchlorid, 12-beta-Hydroxypregnadien 3,20-dion, Helenalin, Indicin, Indicin-N-oxid, Lasiocarpin, Inotodiol, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus (Rapamycin), Rapamycin in Kombination mit Arsen oder Arsenverbindungen oder Komplexen, Somatostatin, Tacrolimus, Roxithromycin, Troleandomycin, Simvastatin, Rosuvastatin, Vinblastin, Vincristin, Vindesin, Thalidomid, Teniposid, Vinorelbin, Tropfosfamid, Treosulfan, Tremozolomid, Thiotepa, Tretinoin, Spiramycin, Umbelliferon, Desacetylvismion A, Vismion A und B, Zeorin, Fasudil.

Als Hilfsstoffe können Lösungsmittel, Zucker, Vitamine, Proteine, Polymere, Oligomere, Kontrastmittel als auch andere physiologisch verträgliche Stoffe eingesetzt werden.

Bei den Kontrastmitteln handelt es sich zumeist um Stoffe, welche Iod, Mangan, Eisen, Lanthan, Cer, Praseodym, Neodym, Samarium, Europium, Gadolinium, Terbium, Dysprosium, Holmium, Erbium, Thulium, Ytterbium und/oder Lutetium enthalten. Als Kontrastmittel können übliche Kontrastmittel für Röntgenaufnahmen, Computertomographie (CT), Kernspintomographie oder Magnetresonanztomographie (MRT) eingesetzt werden.

Des Weiteren sind iodhaltige Kontrastmittel bevorzugt, welche bei der Gefäßdarstellung (Angiographie und Phlebographie) und bei der CT (Computertomographie) verwendet werden. Insbesondere bevorzugt sind Kontrastmittel mit einem 1,3,5-Triiodbenzolkern, Röntgenkontrastmittel, Diatrizoesäure, lothalaminsäure, lotrolan, lodixanol, lopamidol, lohexol, lomeprol, lopromid und lotroxinsäure oder gut oder weniger gut lösliche Salze der betreffenden Säuren.

Eine weitere Klasse von bevorzugten Kontrastmitteln stellen die paramagnetischen Kontrastmittel dar, welche zumeist ein Lanthanoid enthalten. Zu den paramagnetischen Substanzen, die über ungepaarte Elektronen verfügen, zählt z.B. das Gadolinium (Gd³⁺), das insgesamt sieben ungepaarte Elektronen besitzt. Des Weiteren gehören zu dieser Gruppe das Europium (Eu²⁺, Eu³⁺), Dysprosium (Dy³⁺) und Holmium (Ho³⁺). Diese Lanthanoide können auch in chelatisierter Form unter Verwendung von beispielsweise Polyazasäuren, Polycarbonsäuren und insbesondere EDTA, DTPA sowie DOTA als Chelatbildner eingesetzt werden. Beispiele für Gadolinium-haltige Kontrastmittel sind Gadolinium-Diethylentriaminpentaessigsäure (auch bekannt als Gadopentetsäure oder GdDPTA), Gadodiamid, Meglumin-Gadoterat, Gadoteridol und Gadobutrol.

Die erfindungsgemäßen Ballonkatheter mit oder ohne aufgesetzten Stent und mit oder ohne Beschichtung können zur lokalen Behandlung und Prophylaxe von Gefäßerkrankungen sowie zur Behandlung von Gefäßwandveränderungen, die den Blutfluß nicht wesentlich einschränken eingesetzt werden. Insbesondere dienen die erfindungsgemäßen Ballonkatheter zur Behandlung und Prophylaxe von Stenose, Restenose, In-Stent-Stenose, Arteriosklerose.

### Beispiele

### Beispiel 1

Fig. 1 zeigt einen beschichteten Ballon unter selbstexpandierendem Stent.

Das System besteht aus einem Ballonkatheter, der einen beschichteten Ballon mit 5 mm Durchmesser, 100 mm Länge mit 3µg Paclitaxel / mm² Ballonoberfläche trägt. Über dem Ballon befindet sich ein selbstexpandierender Nitinol-Stent (5 mm x 80 mm) im nicht expandierten Zustand, der durch einen vom Benutzer zurückziehbaren Schlauch als Expandierschutz auf einen engen Durchmesser komprimiert wird.

Wenn sich der Ballon mit dem Stent in dem stenotischen Bereich des Blutgefäßes befindet wird zunächst der Schlauch zurückgezogen und damit der Stent freigesetzt. Dieser legt sich an die noch immer verengte Gefäßwand an. Anschließend wird der Ballon mit hohem Druck unter Verwendung eines Kontrastmittels soweit expandiert, dass der ursprüngliche Gefäßdurchmesser erreicht wird. Der Ballon überträgt bei diesem Vorgang den Wirkstoff auf die Gefäßwand. Dadurch wird eine unerwünschte Verdickung der Gefäßwand im Heilungsprozess vermieden und das Gefäß langfristig offen gehalten.

Nach erfolgter Stentplatzierung und Dilatation wird der Katheterballon durch Absaugen des Kontrastmittels und den angelegten Unterdruck wieder deflatiert und zur Erreichung des ursprünglichen Durchmessers kann der Schlauch wieder in distale Richtung über den Katheterballon geschoben werden. Danach wird der Katheterballon aus dem Blutgefäß entfernt.

### Beispiel 2

Der gefaltete Ballon (4.0 x 40 mm) eines mit 7 µg Methotrexat/ mm² Ballonoberfläche beschichteten Balionkatheters wird spiralförmig mit einem PTFE-Band von 10 µm Stärke und 200 µm Breite so umwickelt, dass der Abstand zwischen den Windungen des Bandes 3 mm beträgt. Das Band wird proximal und distal des Ballons fest mit dem Katheterschaft verbunden. Das Band vergrößert den Durchmesser des gefalteten Ballons nicht messbar. Beim Expandieren des Ballons am Ort einer Blutgefäßverengung reißt das Band an beliebiger Stelle ohne dass die Enden den Kontakt zum Katheterschaft verlieren. Sie werden am Ende der Behandlung mit dem Katheter aus dem behandelten Blutgefäß entfernt.

### Beispiel 3

Ein mit einem Arzneimittel beschichteter Ballonkatheter (5 mm Ballondurchmesser, 120 mm Ballonlänge) wird in gefaltetem Zustand in einen PTFE-Schlauch, 1.6 mm äußerer Durchmesser, 1.3 mm innerer Durchmesser, eingeführt, so dass das distale Ende des Ballonkatheters mit der Katheterspitze und der Öffnung des zentralen Kanals des Katheterschafts (zur späteren Aufnahme des Führungsdrahtes) gerade aus dem PTFE-Schlauch herausragt während sich der wirkstoffbeschichtete Ballon vollständig im Lumen des PTFE-Schlauches befindet. Mit einer Injektionsnadel werden 4 µl des Kontrastmittels Ultravist 370 (Bayer Schering Pharma AG, Berlin) entsprechend einer Lösung von 0.77g lopromid/ml von distal in das Lumen zwischen der Wand des PTFE-Schlauches und die Katheterspitze platziert. Der Schlauch wird für 24 Stunden bei 30°C gelagert. Danach findet sich am distalen Ende des Schlauches ein Pfropf aus festem Kontrastmittel, der etwa 3 mm Strecke des PTFE-Schlauches fest verschließt. Das System wird in üblicher Weise mit Ethylenoxid-Gas sterilisiert.

Prüfung auf Wirksamkeit des Verschlusses:
Der Schlauch wird mit dem distalen Ende in auf 40°C erwärmtes Blut getaucht und rhythmisch bewegt. Es dauert etwa 5 Minuten bevor sich der Kontrastmittelpfropf soweit aufgelöst hat, daß Blut in den Schutzschlauch eindringen kann.
Bei Bedarf lässt sich der Kontrastmittelpfropf mit dem Ballonkatheter auch früher aus dem PTFE-Schlauch drücken oder durch Zurückziehen des Katheters oder Schlauches vorzeitig lösen. Befindet sich der Kontrastmittelpfropf außerhalb des Schutzschlauches und ist von allen Seiten von Blut umgeben löst er sich binnen Sekunden bis maximal 1 Minute vollständig auf.

## Patentansprüche

1. Wirkstoffbeschichteter Ballonkatheter umfassend einen Katheterschaft, einen schlauchförmigen Expandierschutz mit Abdichtung des Raumes zwischen Katheterschaft und schlauchförmigen Expandierschutz gegen das Eindringen von Körperflüssigkeiten und einen Katheterballon, wobei der Expandierschutz bis zur Expansion den Katheterballon vor vorzeitiger Aufweitung und vorzeitigem Verlust von Beschichtungsmaterial schützt, wobei der Expandierschutz optional einen selbstexpandierenden Stent enthalten oder umfassen kann,
**dadurch gekennzeichnet dass** der Expandierschutz distal mittels pharmazeutisch und physiologisch unbedenklicher, löslicher Substanzen verschlossen ist.

2. Wirkstoffbeschichteter Ballonkatheter nach Anspruch 1, wobei der Expandierschutz einen darin befindlichen selbstexpandierenden Stent enthält, wobei der Expandierschutz bis zur Freisetzung den Stent vor vorzeitiger Aufweitung schützt.

3. Wirkstoffbeschichteter Ballonkatheter nach Anspruch 1 oder 2, wobei der Expandierschutz am distalen Ende oder am proximalen Ende oder am distalen Ende und am proximalen Ende einen röntgendichten Marker aufweist.

4. Wirkstoffbeschichteter Ballonkatheter nach einem der Ansprüche 1 - 3, wobei es sich bei dem Expandierschutz um einen perforierten oder durchgängigen Schutzschlauch handelt.

5. Wirkstoffbeschichteter Ballonkatheter nach Anspruch 4, wobei der Schutzschlauch von seinem proximalen Ende aus in proximale Richtung bewegbar ist und bei der Bewegung in proximale Richtung den Katheterballon teilweise oder völlig freigesetzt.

6. Wirkstoffbeschichteter Ballonkatheter nach Anspruch 4 oder 5, wobei der Schutzschlauch nach seiner Bewegung in proximale Richtung auch wieder in distale Richtung bewegbar ist und nach der Dilatation den Katheterballon durch Bewegung in distale Richtung wieder umgibt.

7. Wirkstoffbeschichteter Ballonkatheter nach einem der Ansprüche 1 - 6, wobei der Expandierschutz nach der Dilatation des Katheterballons zusammen mit dem Katheterballon wieder aus dem Körper entfernt wird.

8. Wirkstoffbeschichteter Ballonkatheter nach einem der Ansprüche 1 - 7, wobei der Katheterballon einen vormontierten Stent enthält, der nach Expansion des Katheterballons wieder einen geringeren Querschnitt annimmt und mit dem Ballonkatheter aus dem Körper entfernt wird.

9. Wirkstoffbeschichteter Ballonkatheter nach Anspruch 8, wobei der Katheterballon länger ist als der auf dem Katheterballon vormontierte Stent.

10. Wirkstoffbeschichteter Ballonkatheter umfassend einen wirkstofffreien elastischen Expandierschutz und einen Katheterballon, wobei der Expandierschutz bis zur Expansion den Katheterballon vor vorzeitiger Aufweitung schützt und nach erfolgter Expansion die Rückfaltung des Katheterballons unterstützt.

11. Wirkstoffbeschichteter Ballonkatheter nach Anspruch 10, wobei es sich bei dem Expandierschutz um ein flexibles Band, ein flexibles Netz, ein flexibles Gitter, eine flexible Folie oder eine flexible Spirale handelt.

12. Wirkstoffbeschichteter Ballonkatheter nach Anspruch 10 oder 11, wobei das flexible Band, das flexible Netz, das flexible Gitter, die flexible Folie oder die flexible Spirale bei der Expansion des Katheterballons eine entgegengesetzte zur Längsachse des Katheterballons wirkende Kraft ausübt.

13. Wirkstoffbeschichteter Ballonkatheter nach einem der Ansprüche 10-12, wobei das flexible Band, das flexible Netz, das flexible Gitter, die flexible Folie oder die flexible Spirale bei der Deflation des Katheterballons die Rückfaltung des Katheterballons unterstützt und den Katheterballon wieder auf einen Durchmesser bringt, welcher maximal 30% größer, bevorzugt 20% größer, weiter bevorzugt 10% größer und insbesondere bevorzugt 5% größer als der Durchmesser des Katheterballons vor der Inflation des Katheterballons ist.

14. Wirkstoffbeschichteter Ballonkatheter nach einem der Ansprüche 10 oder 11, wobei der Expandierschutz durch einen Stent bewirkt wird, der nach Implantation keine relevante Stützfunktion ausübt.

15. Wirkstoffbeschichteter Ballonkatheter nach einem der Ansprüche 10 - 13, wobei der Expandierschutz in Durchmesser und Länge weitgehend dem zusammengefalteten Katheterballon entspricht.

## Claims

1. A drug-coated balloon catheter comprising a catheter shaft, a tubular expansion protection with a sealing of the space between the catheter shaft and the tubular expansion protection against the penetration of bodily fluids and a catheter balloon, wherein the expansion protection protects against premature expansion and premature loss of coating material up to the expansion of the catheter balloon, wherein the expansion protection can optionally contain or comprise a self-expanding stent,
**characterized in that** the expansion protection is closed distally by pharmaceutically and physiologically safe, soluble substances.

2. The drug-coated balloon catheter according to Claim 1, wherein the expansion protection contains a self-expanding stent in it, wherein the expansion protection protects the stent from premature expansion until the release.

3. The drug-coated balloon catheter according to Claim 1 or 2, wherein the expansion protection has a radiopaque marker on the distal end or on the proximal end or on the distal end and on the proximal end.

4. The drug-coated balloon catheter according to one of Claims 1 - 3, wherein the expansion protection is a perforated or continuous protection tube.

5. The drug-coated balloon catheter according to Claim 4, wherein the protection tube can be moved out from its proximal end in a proximal direction and during the movement in the proximal direction it partially or completely releases the catheter balloon.

6. The drug-coated balloon catheter according to Claim 4 or 5, wherein after its movement in the proximal direction the protection tube can be moved back in the distal direction again and after the dilation it again surrounds the catheter balloon by moving in the distal direction.

7. The drug-coated balloon catheter according to one of Claims 1 - 6, wherein the expansion protection is removed together with the catheter balloon from the body again after the dilation of the catheter balloon.

8. The drug-coated balloon catheter according to one of Claims 1 - 7, wherein the catheter balloon contains a pre-mounted stent which again assumes a smaller cross section after the expansion of the catheter balloon and is removed with the balloon catheter out of the body.

9. The drug-coated balloon catheter according to Claim 8, wherein the catheter balloon is longer than the stent pre-mounted on the catheter balloon.

10. A drug-coated balloon catheter comprising a drug-free, elastic expansion protection and a catheter balloon, wherein the expansion protection protects the catheter balloon from premature expansion up to the expansion of the catheter balloon and supports the refolding of the catheter balloon after the expansion has taken place.

11. The drug-coated balloon catheter according to Claim 10, wherein the expansion protection is a flexible band, a flexible netting, a flexible grid, a flexible film or a flexible spiral.

12. The drug-coated balloon catheter according to Claim 10 or 11, wherein the flexible band, the flexible netting, the flexible grid, the flexible film or the flexible spiral exerts a force acting opposite the longitudinal axis of the catheter balloon during the expansion of the catheter balloon.

13. The drug-coated balloon catheter according to one of Claims 10-12, wherein the flexible band, the flexible netting, the flexible grid, the flexible film or the flexible spiral supports the refolding of the catheter balloon during the deflation of the catheter balloon and brings the catheter balloon back to a diameter which is maximally 30% greater, preferably 20% greater, more preferably 10% greater and especially preferably 5% greater than the diameter of the catheter balloon before the inflation of the catheter balloon.

14. The drug-coated balloon catheter according to one of Claims 10 or 11, wherein the expansion protection is brought about by a stent that does not exert any relevant support function after implantation.

15. The drug-coated balloon catheter according to one of Claims 10 - 13, wherein the expansion protection corresponds largely in diameter and length to the folded catheter balloon.

## Revendications

1. Cathéter à ballonnet à élution médicamenteuse comprenant une tige, une protection anti-dilatation tubulaire disposant d'un joint situé entre la tige du cathéter et la protection anti-dilatation tubulaire et qui empêche les liquides biologiques de pénétrer, et un ballonnet, la protection anti-dilatation évitant la dilatation prématurée du ballonnet et la fuite prématurée du matériau de revêtement avant dilatation du ballonnet, la protection anti-dilatation tubulaire pouvant éventuellement contenir ou comprendre une endoprothèse à dilatation automatique,
**caractérisé en ce que** la protection anti-dilatation est fermée au niveau distal par des substances solubles sans danger du point de vue pharmaceutique et physiologique.

2. Cathéter à ballonnet à élution médicamenteuse selon la revendication 1, la protection anti-dilatation contenant à l'intérieur une endoprothèse à dilatation automatique, la protection anti-dilatation évitant à l'endoprothèse de se dilater prématurément avant sa libération.

3. Cathéter à ballonnet à élution médicamenteuse selon la revendication 1 ou 2, la protection anti-dilatation contenant un marqueur radio-opaque à l'extrémité distale, ou à l'extrémité proximale, ou à l'extrémité distale et à l'extrémité proximale.

4. Cathéter à ballonnet à élution médicamenteuse selon l'une quelconque des revendications 1 à 3, la protection anti-dilatation étant un tube de protection perforé ou continu.

5. Cathéter à ballonnet à élution médicamenteuse selon la revendication 4, le tube de protection pouvant sortir de son extrémité proximale dans le sens proximal, et pendant son déplacement dans le sens proximal, il libère partiellement ou complètement le ballonnet du cathéter.

6. Cathéter à ballonnet à élution médicamenteuse selon la revendication 4 ou 5, le tube de protection pouvant revenir dans le sens distal après son déplacement dans le sens proximal, et une fois la dilatation effectuée, il entoure de nouveau le ballonnet du cathéter en se déplaçant dans le sens distal.

7. Cathéter à ballonnet à élution médicamenteuse selon l'une quelconque des revendications 1 à 6, la protection anti-dilatation étant retirée du corps en même temps que le ballonnet du cathéter après dilatation de celui-ci.

8. Cathéter à ballonnet à élution médicamenteuse selon l'une quelconque des revendications 1 à 7, le ballonnet du cathéter contenant une endoprothèse préalablement montée qui revient à un calibre inférieur après dilatation du ballonnet du cathéter et qui est retirée du corps en même temps que le cathéter à ballonnet.

9. Cathéter à ballonnet à élution médicamenteuse selon la revendication 8, le ballonnet du cathéter étant plus long que l'endoprothèse sur lequel elle est préalablement montée.

10. Cathéter à ballonnet à élution médicamenteuse comprenant une protection anti-dilatation élastique exempte de médicament et un ballonnet, la protection anti-dilatation évitant au ballonnet du cathéter de se dilater prématurément, et contribuant au repliement du ballonnet une fois la dilatation effectuée.

11. Cathéter à ballonnet à élution médicamenteuse selon la revendication 10, la protection anti-dilatation étant une bande souple, un filet souple, une grille souple, un film souple ou une spirale souple.

12. Cathéter à ballonnet à élution médicamenteuse selon la revendication 10 ou 11, la bande souple, le filet souple, la grille souple, le film souple ou la spirale souple exerçant une force contraire à l'axe longitudinal du ballonnet du cathéter pendant la dilatation de celui-ci.

13. Cathéter à ballonnet à élution médicamenteuse selon l'une quelconque des revendications 10 à 12, la bande souple, le filet souple, la grille souple, le film souple ou la spirale souple contribuant au repliement du ballonnet du cathéter pendant le dégonflement de celui-ci et ramenant le ballonnet du cathéter à un diamètre qui est au maximum 30 % supérieur, de préférence 20 % supérieur, de préférence 10 % supérieur, et en particulier de préférence 5 % supérieur au diamètre du ballonnet avant sa dilatation.

14. Cathéter à ballonnet à élution médicamenteuse selon la revendication 10 ou 11, la protection anti-dilatation étant produite par une endoprothèse qui ne joue aucun rôle significatif de soutien après implantation.

15. Cathéter à ballonnet à élution médicamenteuse selon l'une quelconque des revendications 10 à 13, la protection anti-dilatation correspondant largement en diamètre et en longueur au ballonnet du cathéter replié.
